# EUROPEAN PATENT SPECIFICATION

(11) **EP 3 482 680 B1**
(45) Date of publication and mention of the grant of the patent: **19.04.2023**
(21) Application number: 18206169.7
(22) Date of filing: 14.11.2018
(51) Int. Cl.: A61B 5/00

(54) **DEVICE FOR DETECTING IMAGES OF THE MOUTH OF A PATIENT**
VORRICHTUNG ZUR DETEKTION VON BILDERN DES MUNDES EINES PATIENTEN
DISPOSITIF DE DÉTECTION D'IMAGES DE LA BOUCHE D'UN PATIENT

(30) Priority: 14.11.2017 IT 201700130092
(43) Date of publication of application: 15.05.2019
(73) Proprietor: Gaveglia, Vittorio, 04024 Gaeta (LT) (IT)
(72) Inventor: Gaveglia, Vittorio, 04024 Gaeta (LT) (IT)
(74) Representative: Gallo, Luca

(56) References cited:
- WO-A1-2012/090211
- JP-A- 2017 164 399
- US-A- 5 143 086
- US-A1- 2012 263 449

## Description

### Field of application

The present invention regards a device for detecting images of the mouth of a patient.

The present device is advantageously intended to be employed in the general field of cosmetic treatments of the mouth, such as in particular teeth and lips, and in the more particular field of treatment of teeth for clinical or cosmetic purposes and hence in the dental field, in particular for the diagnosis and medical/surgical therapy of pathologies that affect teeth, gums, maxillary bones and temporomandibular joints.

It is an instrument that is applied to the patient during appointments/visits with the professional, in particular a dentist, and it is therefore a dentomaxillofacial orthopedic aid employable for correcting a pathological condition of transverse growth deficit of the upper maxilla which can involve a poor dental occlusion, but which can also have repercussions on speech, on deglutition as well as on respiration.

Therefore, the device for detecting images of the mouth of a patient, object of the present invention, is inserted in the field of dental instruments used by specialized personnel in dental or cosmetic practice.

### State of the art

A device normally termed "facial arch" is known in the dentistry field, and has for some time been employed for the correct stereometric detection of the maxillary position with respect to predefined analysis planes.

The facial arch is employed by the dental professional in order to transfer the patient data to the dental laboratory relative to the exact relation of the two dental arches and to their arrangement in the three spatial planes, so as to be able to correctly mount the plaster models.

Such data is then used by the dental technician due to another instrument termed dental articulator, on which the prosthetic works are then performed.

The articulator allows, due to the transfer of the data actuated by the facial arch, "copying" the movements of the mandible. Indeed, in order to have a successful prosthetic rehabilitation, the dentist must consider factors connected to mandibular biomechanics, which can only be understood by being able to reproduce - by means of the aforesaid instruments (facial arch and articulator) - the static and dynamic relationship of the masticatory system of the patient.

Different facial arch solutions exist on the market. One of the most widespread comprises a curved support which is extended over a substantially horizontal plane and which is set on/abuts at its ends through ear tips in the acoustic meatuses of the patient. In median position, the arch is then connected to a fork holder block comprising a substantially vertical rod, from which the following are extended: a central support which is set on/abuts against a median craniometric point, situated at the root of the nose, on the frontonasal suture in the form of a small depression (commonly termed "nasion") and a fork susceptible of receiving the wax for the impression of the upper dental arch.

In operation, once the facial arch is mounted in abutment against the meatuses and against the nasion, the fork carrying the wax is inserted in the mouth of the patient; at this point, the facial arch is removed from the mouth of the patient after the reference of the upper teeth has been taken. The fork is then separated from the facial arch, mounted on a transfer table in order to be inserted in the articulator, thus reproducing the exact position of the upper dental model of the patient.

The facial arch has the drawback of having a very limited functionality and of requiring a complex series of operations, calibration and fixing of the various components, which can compromise the correct transfer of the data and which in any case require considerable experience. The successful outcome of the data transfer of the upper dental arch of the patient onto the articulator thus depends on the ability of the professional operator who carries out the abovementioned operations.

Facial arches are also known which make use of ultrasound, connected to a computer in order to provide a representation of the dental arches of the patient and a calculation of the parameters for programming the articulator, recording and analyzing all the movements of the patient's mandible.

From DE 1995687, it is in particular known to carry out the precise recording of the state and mobility of the mandible articulation, with the aid of measuring systems based on the travel time of the ultrasound pulses, which allow simple and quick measurements with extremely light sensors.

From the patent US 2016030144, a facial arch is also known which is of digital type, in order to capture the dental-facial characteristics of a patient by acquiring and recording data that define the tilt of the occlusal plane of the teeth of a patient in three spatial planes with respect to the cranium, to the head and/or to the face. The system includes a substrate adapted to support the bite registration material of the patient and a digital inclinometer associated with the substrate in order to register the orientation of the occlusal plane. A platform with adjustable mounting (of the type of the abovementioned articulator) is adapted in order to receive the substrate and replicate the tilt registered by the multiple-axis digital inclinometer.

The above-described facial arch devices of known type are only intended for transferring data of the upper dental arch of the patient to an articulator and are poorly adapted for being used for other purposes.

In addition, the above-described devices do not allow obtaining representations and measurements of the dental arches or of other parts of the mouth of patients in a practical and easy manner, as well as repeatedly over time without introducing errors. Indeed, the devices of known type are complex to fit on the patient and do not allow a comparison of data over time, since if on one hand they use invariant references of the cranium of the patient, such as the nasion and the acoustic meatuses of the patient, on the other hand they require the use of sensors which do not provide invariant absolute data over time, depending in particular on their positioning with respect to the patient. In addition, the data that is obtained from such sensors does not allow comparing images over time with the precision required for the many different needs in the orthodontics field.

The documents WO 2012/090211 and JP 2017164399 describe devices of known type for detecting the mandibular movement of the face, which comprise a video camera with flexible support bands intended to be wound around the head of the patient, nevertheless not allowing a good reference for the images detected by the video camera.

The document US 5,143,086 describes a further device of known type for detecting the mandibular movement of the face, which provides for the use of infrared sensors adapted to detect the trajectories of infrared LEDs attached to the teeth of the patient, nevertheless not allowing the acquisition of complete data regarding the morphology of the mouth or of the teeth.

### Presentation of the invention

In this situation, the problem underlying the present invention is to eliminate the drawbacks of the abovementioned prior art by providing a device for detecting images of the mouth of a patient which is easy and practical for a dentist to use, allowing a quick and easy fitting thereof on the head of the patient.

A further object of the present finding is to provide a device for detecting images of the mouth of a patient which allows detecting images of details of the patent's mouth in a precise and repeatable manner over time, without introducing reference errors.

A further object of the present finding is to provide a device for detecting images of the mouth of a patient which is structurally simple and entirely reliable in operation.

A further object of the present finding is to provide a device for detecting images of the mouth of a patient which is provided with a high mechanical stability.

A further object of the present finding is to provide a device for detecting images of the mouth of a patient which allows obtaining an improved prosthetic reconstruction of the teeth of the patient.

A further object of the present finding is to provide a device for detecting images of the mouth of a patient which allows obtaining evaluations of the articular dynamics of the patient's mandible.

A further object of the present finding is to provide a device for detecting images of the mouth of a patient which allows obtaining teeth with a constant monitoring of the optimal design.

A further object of the present finding is to provide a device for detecting images of the mouth of a patient which allows the patient to appreciate the final effect of a proposed prosthetic intervention, for his/her decision on the matter.

### Brief description of the drawings

The technical characteristics of the invention, according to the aforesaid objects, and the advantages thereof will be more evident from the following detailed description, made with reference to the enclosed drawings, which represent several merely exemplifying and non-limiting embodiments of the invention, in which:

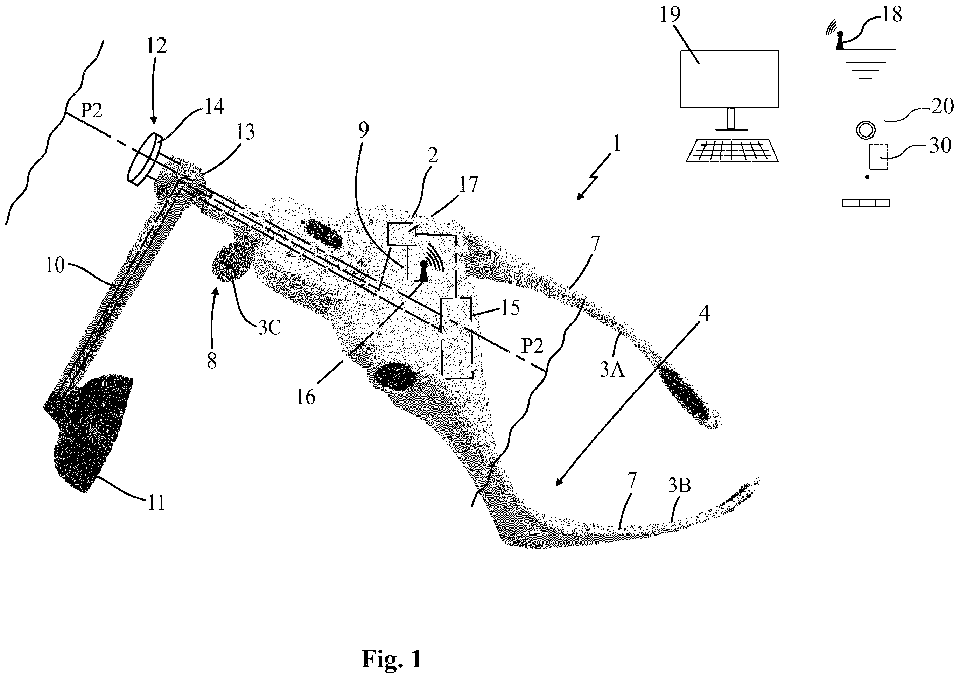

- figure 1 shows a general perspective view of an embodiment of the device for detecting images of the mouth of a patient according to the present invention, with dashed lines indicating the internal circuitry elements of the support frame of the same device;

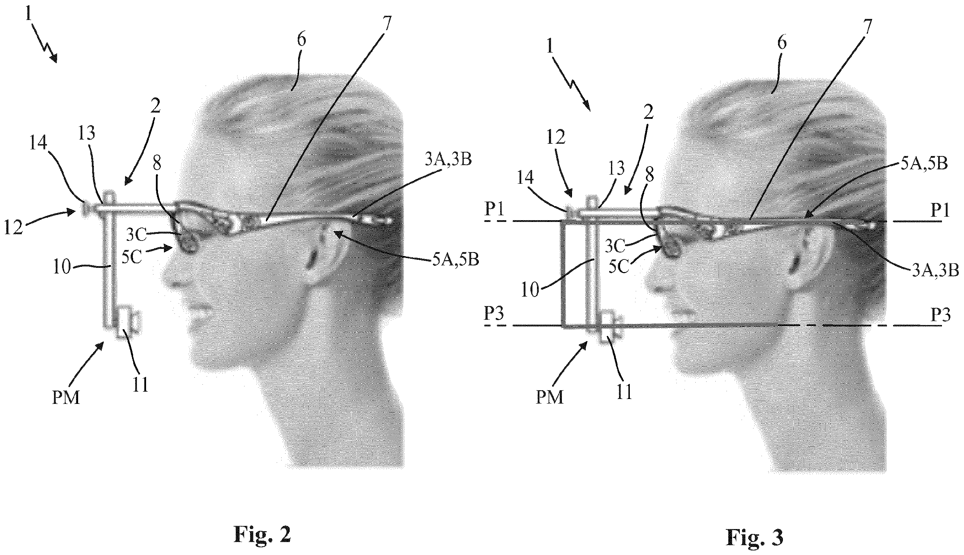

- figure 2 shows a side view of the device of figure 1 worn on the head of a patient;
- figure 3 shows the side view of the device of figure 2, with the reference plane of the head of the patient and the measuring plane of the device indicated;

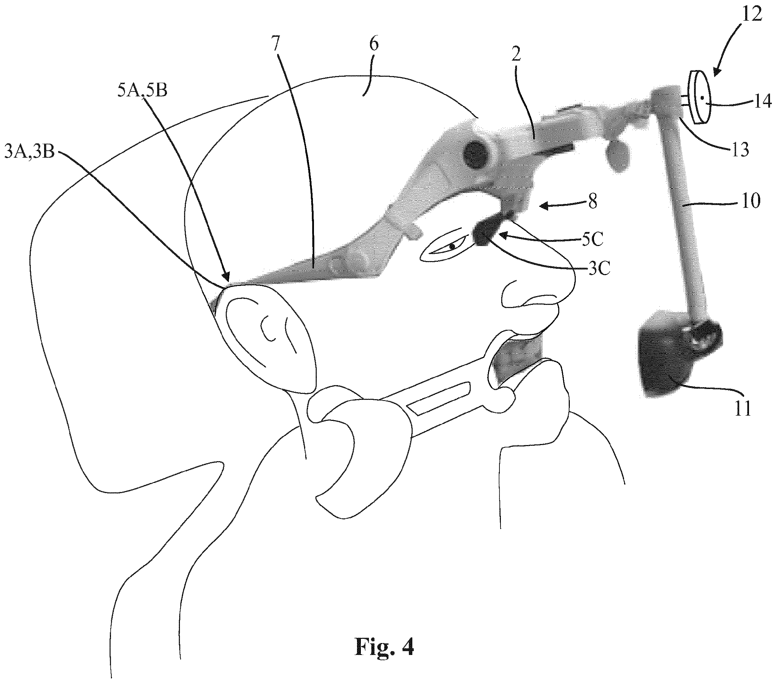

- figure 4 shows a perspective view of the device of figure 1 worn on the head of a patient;
- figures 5-12 show a sequence of steps of a first embodiment of the method for dental reconstruction, object of the present invention;
- figures 13-18 show a sequence of steps of a second embodiment of the method for dental reconstruction, object of the present invention.

### Detailed description of a preferred embodiment

With reference to the enclosed drawings, reference number 1 overall indicates an embodiment of a device for detecting images of the mouth of a patient, object of the present invention.

The device for detecting images, according to the present invention, is intended to be employed in the general field of cosmetic treatments of the mouth such as in particular teeth and lips, and above all in the dental prosthetic field for the design and construction of prostheses and more generally in the dentistry field for medical/surgical diagnosis and therapy of the pathologies that affect teeth, gums, maxillary bones and temporomandibular joints.

This is an instrument which is applied to the patient during the dental appointment/visit by a medical professional, in particular dentist, and for example as will be clarified hereinbelow it allows the patient and dentist to make decisions on prosthetic designs during the appointments themselves or it allows the dentist to be assisted while making the prosthesis in order to obtain reconstructions of improved quality, less subject to the artistic ability of the professional.

Therefore, the device 1 for detecting images of the mouth of a patient, object of the present invention, is inserted in the field of dental instruments for use by personnel specialized in dental or cosmetic practice.

The device 1 comprises a support frame 2 provided with at least three support elements 3A, 3B and 3C defining a seat 4 for the head of patients and intended to be set/abutted against the head of the patient by means of three abutment areas thereof.

The support frame 2 is shaped in order - with these three support elements 3A, 3B and 3C - to be vertically set on/abutted against three corresponding reference points 5A, 5B and 5C of the head 6 of patients identifying a reference plane P1 coinciding with the plane defined by the three abutment areas of the aforesaid three support elements 3A, 3B and 3C of the support frame 2, and such reference plane P1 is substantially unchangeable over time and integral with the head 6 of the patient.

Advantageously, the support frame 2 comprises two lateral abutment rods 7 shaped in order to be, at terminal sections thereof - with two abutment points 3A and 3B - set on/abutted against two corresponding reference points 5A and 5B of the head 6 of the patient arranged in supra-auricular areas. The support frame 2 also comprises a central abutment structure 8 shaped in order to be set/abut - with one end thereof - on/against a reference point 3C of the head 6 of the patient arranged at the nasion.

Advantageously, the lateral abutment rods 7 and the central abutment structure 8 are configured in order to be vertically set on/abutted against the corresponding reference points 5A, 5B, 5C, which in particular also define support points of the support frame 2 on the head 6 of the patient.

More clearly, each time that the support frame 2 is fit on the head 6 of the patient, its three support elements 3A, 3B and 3C find - in the reference points 5A, 5B and 5C of the cranium of the patient - the definition of an invariant reference plane P1, so as to always ensure with each fitting of the support frame 2 an identical positioning thereof with respect to the head 6 of the patient. The supra-auricular areas and the nasion are directly in proximity to cranial bones and therefore they define precise positions over time which do not change over time, and hence in substance an unchangeable plane over time that coincides with the reference plane P1 defined by the areas of abutment of the three support elements 3A, 3B, 3C of the support frame 2 when the device 1 is fit on the head 6 of the patient.

More in detail, the two lateral abutment rods 7 are joined by a front portion 9 of the support frame 2, from which the central abutment structure 8 centrally projects, for example in the form of a nosepiece with two lateral abutments advantageously made of soft material.

In particular, the support frame 2 has a substantially non-pliable structure, such that it can be sufficiently rigid in order to remain in position without being deformed when it is set on/abutted against the reference points 5A, 5B, 5C of the head 6 of the patient. In substance, this allows arranging the device 1 always on the same reference points 5A, 5B, 5C of the same patient. In particular, the lateral abutment rods 7 and the central abutment structure 8 are connected to each other and made of sufficiently rigid material such to define a structurally stable shape of the support frame 2.

The support frame 2 further carries a support rod 10, associated therewith, which is mechanically and projectingly connected to the front portion 9 of the support frame 2.

The support rod 10 is extended, at least for one part thereof, below the reference plane P1 and in particular below the support frame 2.

Preferably, the support frame 2 has a structure similar to an eyeglass frame, with the lateral abutment rods 7 which act as temples and with the central abutment structure 8 which acts as nosepiece of the frame.

Advantageously, such support frame 2, like an eyeglass frame, has a symmetry with respect to a median vertical plane P2 with the nosepiece centered on the symmetry plane.

Advantageously, also the support rod 10 is connected to the support frame 2 at the median plane P2 and preferably is extended from the front portion 9 of the frame 2 orthogonal to the reference plane P1 defined by its three abutment points 3A, 3B and 3C.

The device 1 also comprises a video camera 11, with filming field directed towards the seat 4 defined by the frame 2, according to a measuring plane P3 having a fixed angle with respect to the reference plane P1 and advantageously parallel to the reference plane P1. The video camera 11 is mechanically connected to the support rod 10 in a measuring position PM associable with the patient at a defined distance with respect to the aforesaid reference plane P1 (i.e. in a position defined on the same support rod 10). More in detail, the position of the video camera 11 is defined along the support rod 10 when the measuring plane P3 of the video camera 11 is centered on the mouth or on the detail of the patient's mouth. Such measuring position PM is repeatable for each patient by repositioning the video camera 11 in the same relative position on the support rod 10.

In particular, the video camera PM is arranged below the reference plane P1, and in particular below the support frame 2, such that the filming field of the video camera 11 can be arranged substantially across from the mouth of the head 6 of the patient. Consequently, in particular, also the measuring position PM and the measuring plane P3 are arranged below the reference plane P1.

With the term video camera 11, it must be intended an electronic device capable of taking digital photographs and/or digital video filming.

In particular, the video camera 11 is configured for operating in the visible spectrum and, therefore, is adapted to film images A in the visible light spectrum, e.g. color images or black and white images, or with suitable chromatic filters.

In this manner, the video camera 11 is capable of reproducing images A over time of the mouth or of the detail of the mouth of each patient according to a measuring plane P3 which is always the same in the different measurements and which is in fact always arranged in an identical manner with respect to the reference plane P1, defined in a fixed manner on the head 6 of the patient.

Due to the device 1, object of the present invention, the video camera 11 can be made integral with the head 6 of the patient by means of a support constituted by the support frame 2, which allows filming with images or videos without ever losing the position with respect to the framed subject, that is with respect to the mouth or to the detail of the mouth (e.g. one or more teeth) of the patient.

Upon moving the head 6 of the patient, the video camera 11 maintains its reference on the mouth of the patient unchanged, and the image A that it records is not at all affected by the aforesaid movement.

The reference points 5A, 5B and 5C of the cranium of the patient, i.e. the three abutment points 3A, 3B and 3C of the frame 2, identify a fixed reference plane P1, allowing the repeatability of the photo and video acquisitions and hence allowing the clinic to evaluate the outcomes of conducted dental therapies, through a comparison of images and videos made at different times but in a manner that is exactly repeatable in order to make an optimal comparison.

Due to the above-described device 1, it is thus possible for the dentist to follow - on a display in real time - dental procedures such as carrying out the reconstruction of a tooth directly in the mouth, by executing on a monitor the superimposition of the drawing of the ideal tooth design.

In order to vary the measuring position PM of the video camera 11 as a function of the size of the head 6 of the patient, the device 1 comprises advantageously adjustment means 12 which allow moving the video camera 11 along the support rod 10 in order to define measuring positions PM relative to the head 6 of each patient, i.e. with the video camera 11 centered on the patient's mouth or on the detail of the mouth of each patient.

In accordance with one possible embodiment, the adjustment means 12 for the measuring position PM of the video camera 11 on the support rod 10 comprise a guide 13 integral with the support frame 2 in order to slidably move the support rod 10 with respect to the support frame 2, and blocking means 14, e.g. comprising a knob fixed to a screw which is engaged in a threaded through hole orthogonal to the guide 13, so to be able to interfere by means of the end thereof with the support rod 10 in order to lock it in the pre-selected position with respect to the guide 13 or with respect to the front part of the frame 2 and as a function of the head 6 of the patient. The guide 13 can for example be obtained with a through hole, advantageously with vertical axis, made in a projecting appendage of the frame 2.

In accordance with this embodiment, the video camera 11 is fixed to the support rod 10 for example at the lower end thereof, such that it can adjustably move together with the support rod when the latter slides in the guide 13.

In accordance with a further embodiment not illustrated in the enclosed figures, the device 1 provides for two or more video cameras 11, arranged in corresponding measuring positions that are spaced from each other, with filming field directed towards the seat 4 defined by the frame 2 and susceptible of having orientation towards the mouth of the patient. Advantageously, the two or more video cameras lie on a common measuring plane P3 having fixed angle with respect to the reference plane P1, as well as advantageously parallel to the reference plane P1.

Due to the presence of two or more video cameras, it is also possible to detect the depth of the oral cavity and in particular of teeth and gums for a more complete description/measurement of all the characteristics of the patient's mouth. Due in fact to simple trigonometric relations, the two video cameras can allow detecting the three-dimensionality of the teeth in the mouth of the patient, also detecting the measurements thereof.

The above-described support rod 10 has a graduated scale, for example with centimeters and millimeters indicated in order to always block the support rod 10 with respect to the guide 13 in the same measuring position PM for each patient.

Otherwise, in an equivalent manner and in accordance with an alternative embodiment not illustrated in the enclosed figures, the adjustment means 12 for the measuring position PM of the video camera 11 on the support rod 10 comprise a guide made on a support of the video camera 11 in which the support rod 10 is slidably engaged so as to allow the video camera to slide along the support rod 10 and be locked in position by blocking means, e.g. of the above-described type with the screw that is inserted in a threaded through hole made on the support of the video camera 10.

In accordance with an embodiment variant not illustrated in the enclosed figures, the support rod 10 is extended from a lateral portion of the support frame 2 in order to reach the measuring position PM, which is placed facing the mouth of the patient and hence no longer from above as in the embodiment represented in the enclosed figures, but rather from below, i.e. in part also laterally in order to allow the dentist more freedom of movement in order to more easily operate in the mouth of the patient.

With the term lateral portion of the support frame 2, it is preferably intended at least one portion of at least one of the lateral abutment rods 7 or at least one lateral portion of the front portion of the frame 9.

More in detail, for example, the guide 13 and the blocking means 14 are mechanically fixed on the lateral portion of the support frame 2 and the support rod 10 is slidably connected to the guide 13, being vertically movable with respect to the support frame 2. Due to such adjustment, the video camera 11 can be centered at the measuring point facing the mouth of each patient. In accordance with the aforesaid embodiment variant, the support rod 10 is extended along a path that brings the video camera 11 to be positioned in the aforesaid measuring position, e.g. being formed by at least one lateral portion (preferably vertical) for the adjustment of the height of the video camera as a function of the position of the patient's mouth with respect to the reference plane P1, and a front portion which brings the video camera from the lower end of said lateral portion up to the measuring position. Such front portion can take on different shapes, such as the curved shape or it can be in turn formed by multiple sub-portions.

The device 1 also comprises an electrical power source, in particular a battery 15, mounted in a seat of the support frame 2 in order to power supply the video camera 11.

Otherwise the electrical power source can be represented by a power supply connected to the power grid and connected to the video camera 11 by means of a transport cable.

Advantageously, the device 1 comprises a logic control unit 17 mounted on the support frame 2 and operatively connected to the video camera 11 in order to receive the images A acquired by the latter. In addition, the device 1 comprises a transmission unit 16, also mounted on the support frame 2 and operatively connected to the logic control unit 17 in order to remotely transmit the images A to a remote reception unit 18. The latter is adapted to receive the images A from the transmission unit 16 and is intended to be applied in a computer 20 in order to allow the latter to display such images A on its monitor 19.

The transmission unit 16 and the reception unit 18 can comprise a data transport cable or preferably a wireless communication and data exchange system of the type per se well-known to the man skilled in the art and thus not presently described in detail.

The logic control unit 17 and the transmission unit 16 can be arranged in corresponding seats made in the support frame 2 or they can also be incorporated in the same video camera 11 mounted on the support structure 2 by means of the support rod 10.

Described hereinbelow is a method for dental reconstruction which employs the above-described device 1 for detecting images of the mouth of a patient; for the sake of simplicity, the same reference numbers used above will be maintained hereinbelow.

Such method provides for putting the aforesaid device 1 on the head of the patient with the three support elements 3A, 3B and 3C in abutment against three corresponding reference points 5A, 5B and 5C of the head 6 of the patient, identifying a reference plane P1 coinciding with the plane defined by the aforesaid three support elements 3A, 3B and 3C of the support frame 2, and such plane P1 is substantially unchanged over time and integral with the head 6 of the patient.

When the device 1 is worn, the two lateral abutment rods 7 are set on/abut against supra-auricular areas 5A, 5B of the head of the patient and the central abutment structure 8 is set on/abuts against the nasion point of the head 6 of the patient.

At this point, a step is provided for adjusting the position of the video camera 11 along the support rod 10 by means of adjustment means 12 in order to move it into measuring position PM aligned with the mouth of the patient.

The adjustment of the video camera along the support rod must be intended both literally - since it is the video camera 11 that is moved in a guided manner on the support rod 10 - and in an equivalent sense, since it is the support rod 10 - to which the video camera 11 is integrally fixed in this case - to be moved with respect to the frame 2 as in the case represented in the enclosed figures.

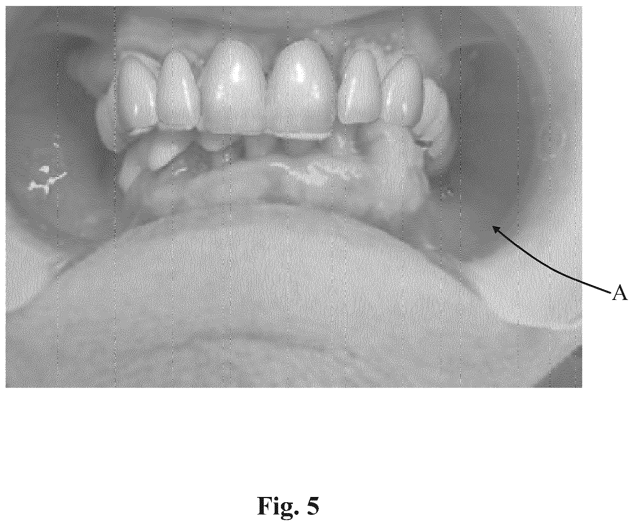

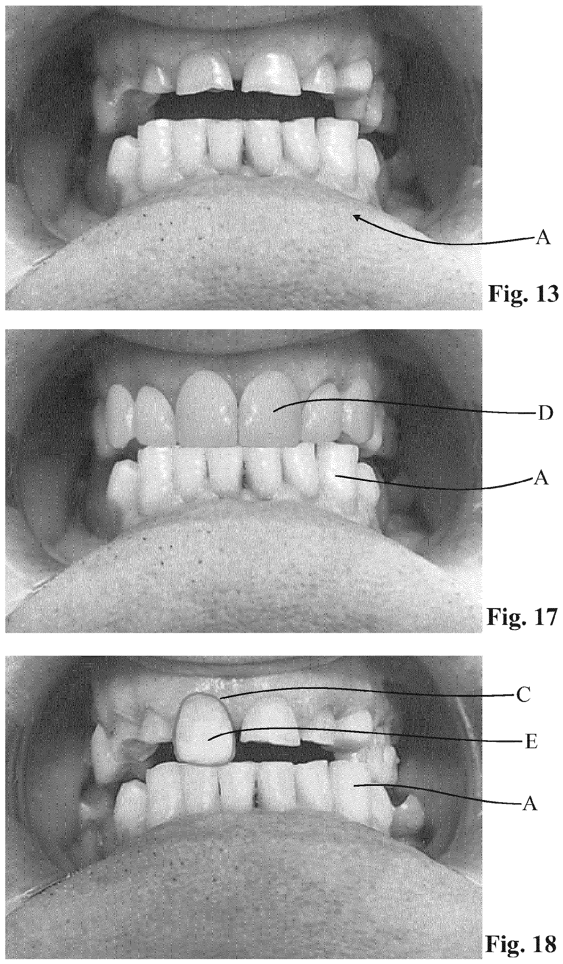

At this point, it is possible to turn on the video camera 11 and take/film therewith - through at least one image A - at least one portion of at least one dental arch of the patient (see figures 5 and 13). In other words, this means taking photographs or filming videos of the state of the teeth of the patient before the prosthetic operation that it is desired to make. Hereinbelow, we will assume that an entire dental arch of the patient, e.g. the upper arch, is filmed.

The image A of the dental arch of the patient is then displayed on the monitor 19 of the computer 20 through a display program so to be able to be examined by the dentist.

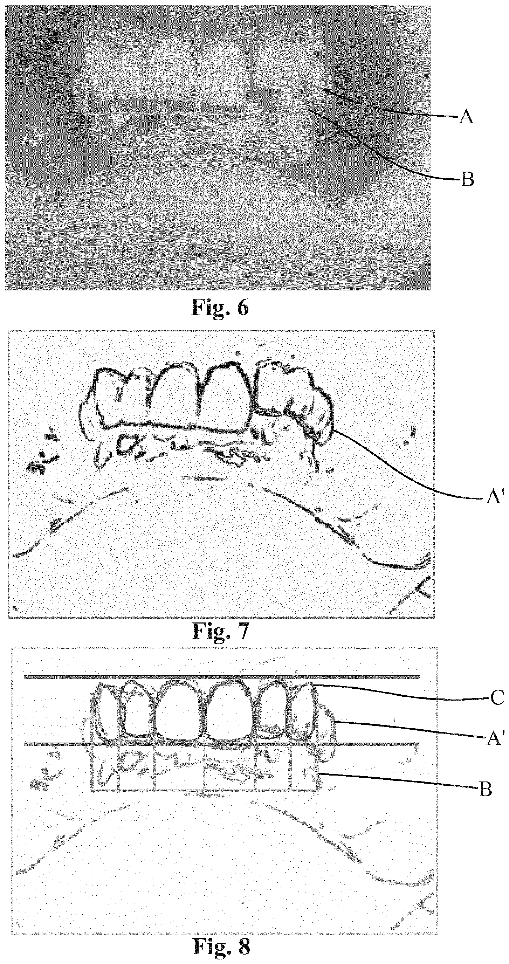

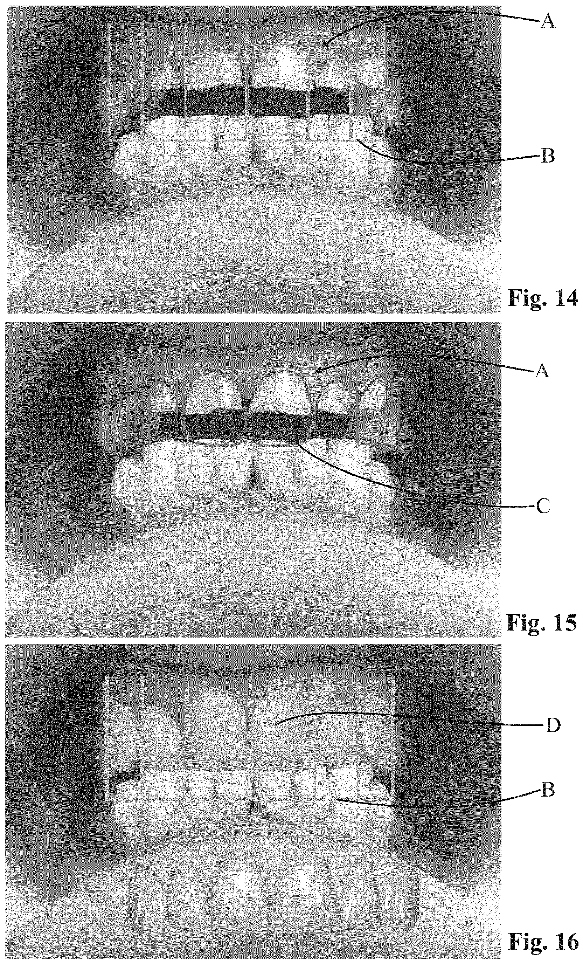

Through a simple image processing program, the dentist can then superimpose on the image A of the dental arch of the patient the image B of a symmetry grid (see figures 6 and 14). This allows the dentist to carry out evaluations regarding the position and/or proportions and/or shapes and/or spacing of the teeth of the arch of the patient.

Advantageously it is preferable to apply a drawing filter for automatically generating the borders A' preferably before superimposing the image of the grid B on the image A of the teeth of the patient (see figure 7). Such filter is present in the image modification software (of Photoshop^{®} type).

Subsequently, the method provides for applying, on the image B of the symmetry grid, the image C of an ideal teeth design, arranging them contained within the margins of the symmetry grid B. During such operation, advantageously, the ideal teeth C are in any case adapted to the mouth of the subject by following the symmetry grids B (see figures 8 and 15).

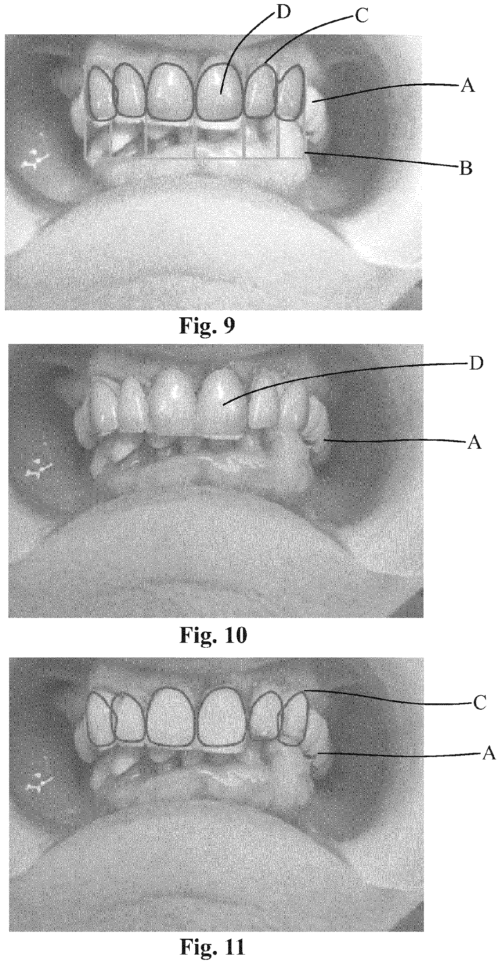

Preferably, it is possible to provide for adding, to the drawing of the ideal teeth C, the template D of the ideal teeth contained in the software program (see figures 9 and 16) and then hiding the symmetry lines B and the ideal teeth C in order to evaluate the functionality of the simulation (see figures 10 and 17).

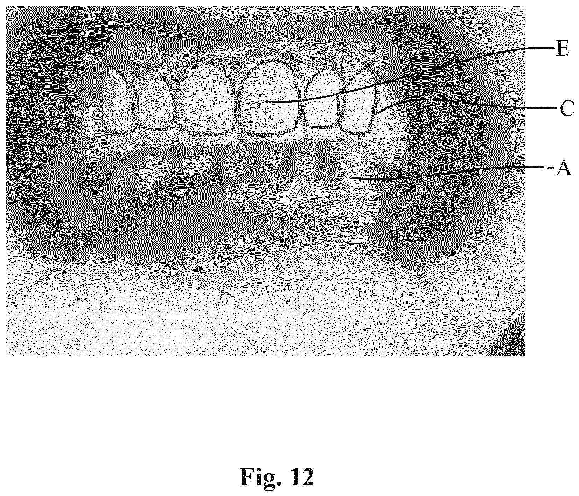

At this point, the dentist can define operations on the teeth of the patient by following, on the monitor, the images of the teeth A that he/she receives from the video camera 11 of his work, e.g. continuously through a video, maintaining the image of the ideal teeth drawings C as reference for the reconstruction of the actual teeth A (see figure 11). In particular, by superimposing the ideal drawing C as guide after the insertion of the temporary resin prosthetic manufactured item in the mouth E, the dentist can easily adapt the shape of the prosthesis to the ideal drawing (see figures 12 and 18).

Since the filming field of the video camera 11 is integral with the head 6 of the patient, it will not be moved with the movement of the patient's head, allowing the dentist to maintain the images of the actual teeth and ideal designed teeth perfectly collimated.

Advantageously, the step of achieving operations on the teeth of the patient by following the drawings of the ideal teeth comprises the reconstruction of at least one tooth, by verifying on the monitor the correspondence between the actual reconstruction of the tooth and the ideal drawing of the tooth.

Preferably, the dentist will take a photograph for each of the above-listed passages so as to archive the procedure carried out for each patient, since useful information might be gathered therefrom at a later date.

In order to implement the above-described method, the present device 1 is provided with a processing module 30 intended to be applied to the computer 20, and obtained for example by means of a software application installed in the computer 20 itself.

Such processing module 30 is configured for superimposing the symmetry grid B on the image A depicting the teeth of the dental arch to be operated (which can also correspond with the temporary resin prosthetic manufactured item E). In particular, the symmetry grid B is provided with multiple reference margins, each delimiting a corresponding area, e.g. with rectangular shape. The processing module 30 arranges the teeth of the image A at the corresponding reference margins of the symmetry grid B, in particular substantially within such reference margins.

Advantageously, the processing module 30 is configured for generating the borders A' of the teeth of the image A before superimposing the symmetry grid B on the latter, as described above.

In addition, the processing module 30 is adapted to apply, to the superimposition of the image A and of the symmetry grid B, a reference image C containing a drawing of an arch of ideal teeth which one desires to obtain. In particular, the ideal teeth of the reference image C are arranged in the corresponding reference margins of the symmetry grid B such to be arranged in front of the corresponding teeth of the image A.

Such superimpositions are advantageously displayed on a screen, such as the monitor 19 of the computer 20.

This allows the dentist to operate on the patient with reference to the video images, as previously described.

The invention as defined therefore achieves the pre-established objects.

## Claims

1. Device (1) for detecting images of the mouth of a patient, comprising:
- a support frame (2) comprising at least three support elements (3A, 3B, 3C) defining a seat (4) for a head (6) of a patient; wherein said three support elements (3A, 3B, 3C) are shaped in order to be set on/abut against three corresponding reference points (5A, 5B, 5C) of the head (6) in a manner such to identify a reference plane (P1) intended to pass through said reference points (5A, 5B, 5C);
- a support rod (10), which is mechanically and projectingly connected to said support frame (2) and is extended at least below said reference plane (P1);
- at least one video camera (11), which is mechanically supported by said support rod (10) below said reference plane (P1) and has a filming field directed towards said seat (4) and oriented with a measuring plane (P3) having a fixed angle with respect to said reference plane (P1);
wherein said video camera (11) is arranged, below said reference plane (P1), in at least one measuring position (PM) placed at a defined distance with respect to said reference plane (P1) and associable with the patient such that it can be substantially centered on the mouth of the patient or on a detail of the patient's mouth,
wherein said video camera (11) is configured for operating in the visible spectrum and for filming, over time, images (A) of the mouth or of the detail of the patient's mouth that are fixed with respect to said reference plane (P1) and to said measuring plane (P3);
wherein said three support elements (3A, 3B, 3C) of said support frame (2) comprise:
- two lateral abutment rods (7) shaped in order to be set on/abut against two reference points (5A, 5B) of the head (6) of the patient arranged in two corresponding supra-auricular areas;
- a central abutment structure (8) shaped in order to be set on/abut against at least one reference point (SC) of the head (6) of the patient arranged at the nasion;
wherein said lateral abutment rods (7) and said central abutment structure (8) are configured in order to be vertically set on/abutted against the corresponding said reference points (5A, 5B, 5C), which define the support points of said support frame (2) on the head (6) of the patient;
wherein said two lateral abutment rods (7) are joined by a front portion (9) of said support frame (2), from which front portion (9) said central abutment structure (8) centrally projects in the form of a nosepiece with two lateral abutments;
wherein said support frame (2) has a substantially non-pliable structure, such that said support frame (2) is sufficiently rigid to remain in position without being deformed when it is set on/abutted against the reference points (5A, 5B, 5C) of the head (6) of the patient;
wherein said device (1) also comprises:
- an electrical power source (15) mounted in a seat of said support frame (2);
- a logic control unit (17) mounted on said support frame (2) and operatively connected to said video camera (11) in order to receive said images (A) from said video camera (11);
- a transmission unit (16) mounted on said support frame (2), operatively connected to said logic control unit (17) and configured for remotely transmitting said images (A);
- a reception unit (18), placed in a remote position with respect to said transmission unit (17), adapted to receive said images (A) from said transmission unit (16) and intended to be applied in a computer (20) in order to display said images (A) on a monitor (19) of the computer (20).

2. Device (1) according to claim 1, which also comprises adjustment means (12) adapted to adjust the measuring position (PM) of said video camera (11) on said support rod (10) and configured for defining measuring positions (PM) relative to the head (6) of each patient and able to be centered as a function of the shape of the head (6) of the patient on his/her mouth or on the detail of his/her mouth.

3. Device (1) according to claim 2, wherein said adjustment means (12) comprise a guide (13) integral with said support frame (2), in order to slidably move said support rod (10) carrying said video camera (11) fixed thereto, with respect to said support frame (2).

4. Device (1) according to claim 2, wherein said adjustment means (12) comprise a guide configured for slidably moving said video camera (11) along said support rod (10).

5. Device (1) according to claim 1, wherein said support frame (2) has a symmetry with respect to a median vertical plane (P2); wherein said support rod (10) is connected to said support frame (2) at said median plane (P3).

6. Device (1) according to claim 1, wherein said support rod (10) is extended from a lateral portion of said support frame (2) in order to reach said measuring portion (PM).

7. Device (1) according to claim 1, which also comprises a processing module (30) intended to be applied to the computer (20) and configured for:
- superimposing a symmetry grid (B) provided with reference margins on said image (A) comprising multiple teeth of a dental arch; wherein said teeth are arranged at respective said reference margins of said symmetry grid (B);
- applying a reference image (C) of an arch of ideal teeth to said symmetry grid (B), arranging said ideal teeth in respective said reference margins of said symmetry grid (B).

8. Device (1) according to claim 7, wherein said processing module (30) is configured for generating borders (A') of the teeth of said image (A) before superimposing said symmetry grid (B) on said image (A).

## Patentansprüche

1. Vorrichtung (1) zur Detektion von Bildern des Mundes eines Patienten, umfassend:
- ein Kopfgestell (2), umfassend mindestens drei Stützelemente (3A, 3B, 3C), die einen Sitz (4) für einen Kopf (6) eines Patienten definieren; bei dem die genannten drei Stützelemente (3A, 3B, 3C) so geformt sind, dass sie an drei entsprechenden Referenzpunkten (5A, 5B, 5C) des Kopfes (6) anliegen/anschlagen, um eine Referenzebene (P1) zu identifizieren, die durch die genannten Referenzpunkte (5A, 5B, 5C) verlaufen soll;
- einen Stützstab (10), der mechanisch und vorstehend mit dem genannten Kopfgestell (2) verbunden ist und mindestens unter der genannten Referenzebene (P1) verläuft;
- mindestens eine Videokamera (11), die mechanisch von dem genannten Stützstab (10) unter der genannten Referenzebene (P1) getragen wird und einen zu dem genannten Sitz (4) gerichteten Aufnahmebereich aufweist und mit einer Messebene (P3) mit einem festen Winkel im Verhältnis zu der genannten Referenzebene (P1) ausgerichtet ist;
wobei die genannte Videokamera (11) unter der genannten Referenzebene (P1) in mindestens einer Messposition (PM) angeordnet ist, die in einem festgelegten Abstand im Verhältnis zu der genannten Referenzebene (P1) positioniert ist, und mit dem Patienten so verbunden werden kann, dass sie im Wesentlichen auf dem Mund des Patienten oder einem Detail des Mundes des Patienten zentriert werden kann,
wobei die genannte Videokamera (11) darauf ausgelegt ist, im sichtbaren Spektrum zu arbeiten und im Laufe der Zeit Bilder (A) des Mundes oder von Details des Mundes des Patienten aufzunehmen, die im Verhältnis zu der genannten Referenzebene (P1) und zu der genannten Messebene (P3) fixiert sind;
wobei die genannten Stützelemente (3A, 3B, 3C) des genannten Kopfgestells (2) Folgendes umfassen:
- zwei seitliche Stützbügel (7), die so geformt sind, dass sie an zwei Referenzpunkten (5A, 5B) des Kopfes (6) des Patienten anliegen/anschlagen, die in zwei entsprechenden Bereichen über der Ohrmuschel angeordnet sind;
- eine mittige Stützstruktur (8), die sie geformt ist, dass sie an mindestens einem Referenzpunkt (SC) des Kopfes (6) des Patienten anliegt/anschlägt, der am Nasion angeordnet ist;
wobei die genannten seitlichen Stützbügel (7) und die genannte mittige Stützstruktur (8) darauf ausgelegt sind, vertikal an den entsprechenden Referenzpunkten (5A, 5B, 5C) anzuliegen/anzuschlagen, die die Auflagepunkte des genannten Kopfgestells (2) auf dem Kopf (6) des Patienten definieren;
wobei die genannten zwei seitlichen Stützbügel (7) durch ein Frontteil (9) des genannten Kopfgestells (2) zusammengefügt werden, wobei von diesem Frontteil (9) die genannte mittige Stützstruktur (8) mittig in der Form eines Nasenteils mit zwei seitlichen Stützen vorsteht;
wobei das genannte Kopfgestell (2) im Wesentlichen eine nicht biegbare Struktur aufweist, so dass das genannte Kopfgestell (2) ausreichend steif ist, um in Position zu bleiben, ohne sich zu verformen, wenn es an den Referenzpunkten (5A, 5B, 5C) des Kopfes (6) des Patienten anliegt/anschlägt;
wobei die genannte Vorrichtung (1) außerdem Folgendes umfasst:
- eine elektrische Stromquelle (15), die in einem Sitz des genannten Kopfgestells (2) montiert ist;
- eine logische Steuerung (17), die auf dem genannten Kopfgestell (2) montiert und operativ mit der genannten Videokamera (11) verbunden ist, um die genannten Bilder (A) von der genannten Videokamera (11) zu empfangen;
- ein Übertragungsgerät (16), das auf dem genannten Kopfgestell (2) montiert, operativ mit der genannten logischen Steuerung (17) verbunden und für die Fernübertragung der genannten Bilder (A) ausgelegt ist;
- ein Empfangsgerät (18), das im Verhältnis zu dem genannten Übertragungsgerät (17) in einer entfernten Position platziert und geeignet ist, die genannten Bilder (A) von dem genannten Übertragungsgerät (16) zu empfangen und dazu bestimmt ist, in einem Computer (20) eingesetzt zu werden, um die genannten Bilder (A) auf einem Monitor (19) des Computers (20) anzuzeigen.

2. Gerät (1) nach Anspruch 1, das außerdem Einstellmittel (12) umfasst, die geeignet sind, die Messposition (PM) der genannten Videokamera (11) auf dem genannten Stützstab (10) einzustellen und darauf ausgelegt ist, Messpositionen (PM) in Bezug auf den Kopf (6) jedes Patienten zu definieren und geeignet ist, abhängig von der Form des Kopfes (6) des/der Patienten/in auf seinem/ihrem Mund oder einem Detail seines/ihres Mundes zentriert zu werden.

3. Gerät (1) nach Anspruch 2, bei dem die genannten Einstellmittel (12) eine Führung (13) als Bestandteil des genannten Kopfgestells (2) umfassen, um den genannten Stützstab (10), der die genannten daran befestigte Videokamera (11) trägt, verschiebbar im Verhältnis zu dem genannten Kopfgestell (2) zu bewegen.

4. Gerät (1) nach Anspruch 2, bei dem die genannten Einstellmittel (12) eine Führung umfassen, die darauf ausgelegt ist, die genannte Videokamera (11) verschiebbar entlang des genannten Stützstabs (10) zu bewegen.

5. Gerät (1) nach Anspruch 1, bei dem das genannte Kopfgestell (2) eine Symmetrie im Verhältnis zu einer vertikalen Medianebene (P2) aufweist; wobei der genannte Stützstab (10) mit dem genannten Kopfgestell (2) an der genannten Medianebene (P3) verbunden ist.

6. Gerät (1) nach Anspruch 1, bei dem der genannte Stützstab (10) von einem Seitenabschnitt des genannten Kopfgestells (2) aus verläuft, um die genannte Messposition (PM) zu erreichen.

7. Gerät (1) nach Anspruch 1, das außerdem ein Verarbeitungsmodul (30) umfasst, das dazu bestimmt ist, mit dem Computer (20) angewendet zu werden und auf Folgendes ausgelegt ist:
- Überlagern eines Symmetrierasters (B), das mit Referenzrändern auf dem genannten Bild (A) versehen ist, das mehrere Zähne eines Zahnbogens umfasst; wobei die genannten Zähne an entsprechenden genannten Referenzrändern des genannten Symmetrierasters (B) angeordnet sind;
- Anwenden eines Referenzbildes (C) eines Bogens idealer Zähne auf das genannte Symmetrieraster (B), indem die genannten idealen Zähne in entsprechenden genannten Referenzrändern des genannten Symmetrierasters (B) angeordnet werden.

8. Gerät (1) nach Anspruch 7, bei dem das genannte Verarbeitungsmodul (30) darauf ausgelegt ist, Ränder (A') der Zähne des genannten Bildes (A) zu generieren, bevor das genannte Symmetrieraster (B) das genannte Bild (A) überlagert.

## Revendications

1. Dispositif (1) de détection d'images de la bouche d'un patient, comprenant :
- un cadre de support (2) comprenant au moins trois éléments de support (3A, 3B, 3C) définissant un siège (4) pour la tête (6) d'un patient ; dans lequel lesdits trois éléments de support (3A, 3B, 3C) sont formés de manière à être réglés sur/venir en butée contre trois points de référence correspondants (5A, 5B, 5C) de la tête (6) afin d'identifier un plan de référence (P1) destiné à passer à travers lesdits points de référence (5A, 5B, 5C) ;
- une tige de support (10), qui est reliée mécaniquement et de manière saillante audit cadre de support (2) et est étendue au moins sous ledit plan de référence (P1) ;
- au moins une caméra vidéo (11), qui est supportée mécaniquement par ladite tige de support (10) sous ledit plan de référence (P1) et qui a un champ de vision orienté vers ledit siège (4) et orienté avec un plan de mesure (P3) ayant un angle fixe par rapport audit plan de référence (P1) ;
dans lequel ladite caméra vidéo (11) est disposée, sous ledit plan de référence (P1), dans au moins une position de mesure (PM) placée à une distance définie par rapport audit plan de référence (P1) et qui peut être associée au patient de manière à être sensiblement centrée sur la bouche d'un patient ou sur un détail de la bouche d'un patient,
dans lequel ladite caméra vidéo (11) est configurée pour fonctionner dans le spectre visible et pour filmer, dans le temps, des images (A) de la bouche ou du détail de la bouche du patient qui sont fixes par rapport audit plan de référence (P1) et audit plan de mesure (P3) ;
dans lequel lesdits trois éléments de support (3A, 3B, 3C) dudit cadre de support (2) comprennent :
- deux tiges de butée latérales (7) formées de manière à être réglées sur/venir en butée contre deux points de référence (5A, 5B) de la tête (6) du patient disposés dans deux zones supra-auriculaires correspondantes ;
- une structure de butée centrale (8) formée de manière à être réglée sur/venir en butée contre au moins un point de référence (5C) de la tête (6) du patient disposé au niveau du point nasal ;
dans lequel lesdites tiges de butée latérales (7) et ladite structure de butée centrale (8) sont configurées pour être réglées verticalement sur/venir en butée contre lesdits points de référence correspondants (5A, 5B, 5C), qui définissent les points de support dudit cadre de support (2) sur la tête (6) du patient ;
dans lequel lesdites deux tiges de butée latérales (7) sont unies par l'intermédiaire d'une partie frontale (9) dudit cadre de support (2), ladite structure de butée centrale (8) faisant saillie centralement de ladite partie frontale (9) sous forme d'un embout avec deux butées latérales ;
dans lequel ledit cadre de support (2) présente une structure sensiblement non pliable, de sorte que ledit cadre de support (2) soit suffisamment rigide pour rester en place sans être déformé lorsqu'il est réglé sur/vient en butée contre les points de référence (5A, 5B, 5C) de la tête (6) du patient ;
dans lequel ledit dispositif (1) comprend également :
- une source d'alimentation électrique (15) montée dans un siège dudit cadre de support (2) ;
- une unité de contrôle logique (17) montée sur ledit cadre de support (2) et reliée fonctionnellement à ladite caméra vidéo (11) afin de recevoir lesdites images (A) de ladite caméra vidéo (11) ;
- une unité de transmission (16) montée sur ledit cadre de support (2), reliée fonctionnellement à ladite unité de contrôle logique (17) et configurée pour transmettre à distance lesdites images (A) ;
- une unité de réception (18), placée dans une position éloignée par rapport à ladite unité de transmission (17), adaptée pour recevoir lesdites images (A) de ladite unité de transmission (16) et destinée à être appliquée dans un ordinateur (20) afin d'afficher lesdites images (A) sur un moniteur (19) de l'ordinateur (20).

2. Dispositif (1) selon la revendication 1, qui comprend également des moyens de réglage (12) adaptés pour régler la position de mesure (PM) de ladite caméra vidéo (11) sur ladite tige de support (10) et configurés pour définir des positions de mesure (PM) par rapport à la tête (6) de chaque patient et pouvant être centrés en fonction de la forme de la tête (6) du patient sur la bouche de celui-ci ou sur le détail de la bouche de celui-ci.

3. Dispositif (1) selon la revendication 2, dans lequel lesdits moyens de réglage (12) comprennent un guide (13) intégré audit cadre de support (2), afin de déplacer de manière coulissante ladite tige de support (10) portant ladite caméra vidéo (11) fixée sur celle-ci, par rapport audit cadre de support (2).

4. Dispositif (1) selon la revendication 2, dans lequel lesdits moyens de réglage (12) comprennent un guide configuré pour déplacer de manière coulissante ladite caméra vidéo (11) le long de ladite tige de support (10).

5. Dispositif (1) selon la revendication 1, dans lequel ledit cadre de support (2) présente une symétrie par rapport à un plan vertical médian (P2) ; dans lequel ladite tige de support (10) est reliée audit cadre de support (2) au niveau dudit plan médian (P3).

6. Dispositif (1) selon la revendication 1, dans lequel ladite tige de support (10) est étendue d'une partie latérale dudit cadre de support (2) afin d'atteindre ladite partie de mesure (PM).

7. Dispositif (1) selon la revendication 1, qui comprend également un module de traitement (30) destiné à être appliqué à l'ordinateur (20) et configuré pour :
- superposer une grille symétrique (B) pourvue de marges de référence sur ladite image (A) comprenant plusieurs dents d'une arcade dentaire ; dans lequel lesdites dents sont disposées au niveau desdites marges de référence respectives de ladite grille symétrique (B) ;
- appliquer une image de référence (C) d'une arcade de dents idéales à ladite grille symétrique (B), en disposant lesdites dents idéales dans lesdites marges de référence respectives de ladite grille symétrique (B).

8. Dispositif (1) selon la revendication 7, dans lequel ledit module de traitement (30) est configuré pour générer des limites (A') des dents de ladite image (A) avant de superposer ladite grille symétrique (B) sur ladite image (A).
